**Europäisches Patentamt**

**European Patent Office**

**Office européen des brevets**

(19)

(11) Veröffentlichungsnummer: **0 300 348 B1**

# (12) EUROPÄISCHE PATENTSCHRIFT

(45) Veröffentlichungstag der Patentschrift: **04.12.91**

(51) Int. Cl.⁵: **C02F 3/28**, B01J 8/22

(21) Anmeldenummer: **88111209.8**

(22) Anmeldetag: **13.07.88**

Die Akte enthält technische Angaben, die nach
dem Eingang der Anmeldung eingereicht wurden
und die nicht in dieser Patentschrift enthalten sind.

(54) **Biogasreaktor.**

(30) Priorität: **18.07.87 DE 3723851**

(43) Veröffentlichungstag der Anmeldung:
**25.01.89 Patentblatt 89/04**

(45) Bekanntmachung des Hinweises auf die
Patenterteilung:
**04.12.91 Patentblatt 91/49**

(84) Benannte Vertragsstaaten:
**AT BE CH DE ES FR GB GR IT LI LU NL SE**

(56) Entgegenhaltungen:
**EP-A- 0 046 313**
**EP-A- 0 142 307**
**EP-A- 0 193 969**
**DE-A- 3 326 879**

(73) Patentinhaber: **Märkl, Herbert, Prof. Dr.-Ing.**
**Kleckener Kirchweg 27**
**W-2105 Seevetal 1(DE)**

(72) Erfinder: **Märkl, Herbert, Prof. Dr.-Ing.**
**Kleckener Kirchweg 27**
**W-2105 Seevetal 1(DE)**

(74) Vertreter: **Schmidt-Bogatzky, Jürgen, Dr. Ing.**
**Huth, Dietrich & Partner**
**Warburgstrasse 50**
**W-2000 Hamburg 36(DE)**

EP 0 300 348 B1

**Beschreibung**

Die Erfindung betrifft einen Biogasreaktor, dessen Gehäuse ganz oder teilweise mit aktiver Biomasse gefüllt ist, in dem mittels eines oder mehrerer säulenartig übereinander angeordneter sich jeweils nach unten erweiternder trichterförmiger Trennelemente in deren Reaktorabschnitten jeweils ein Gassammelraum zum Sammeln der aus der Gärsuspension aufsteigenden Gase und ein gasblasenarmer Sedimentationsraum zur Sedimentation aktiver Biomasse-Schlammteilchen ausgebildet ist, in den eine Leitung mit einem Drosselorgan zur Gasabführung geführt ist, mit einer am trichterförmigen Trennelement ausgebildeten Überströmkante und einem an der Trennelementspitze angeordneten rohrartigen Kanalstück, das sich bis in den oberhalb des trichterförmigen Trennelements befindlichen Reaktorraum erstreckt.

In Biogasreaktoren werden organische Substanzen durch die Einwirkung von Mikroorganismen als aktive Biomasse in $CO_2$ und Methan als Biogas umgesetzt. Bei hochbauenden Reaktoren, die aufgrund ihres geringen Bedarfs an Grundfläche technisch anzustreben sind, akkumuliert mit zunehmender Höhe das aufsteigende Biogas. Infolge des dabei entstehenden hohen Gasvolumenstroms wird durch Flotation aktive Biomasse in die oberen Reaktorzonen transportiert und aus dem Reaktor ausgetragen. Um die Abscheideleistung des Reaktors zu erhalten, ist es deshalb notwendig, den Gasvolumenstrom zu begrenzen und Biogas schon in tieferen Reaktorzonen zu entnehmen. Gleichzeitig ist anzustreben, die Strömung so zu führen, daß in den verschiedenen Reaktorhöhen Sedimentationszonen entstehen. Dies soll nach der DE-C- 33 26 879 durch einen Biogasreaktor der eingangs genannten Art erzielt werden. Bei diesem bekannten Biogasreaktor werden zwei formgleiche, kegelförmig nach unten geöffnete Trichter in kleinem Abstand übereinander angeordnet. Im unteren Trichter, der nach oben geschlossen ist, wird das in kleinen Blasen aufsteigende Gas gesammelt und aufgestaut. Aus diesem Gassammelraum kann Gas entnommen und nach außen abgeführt werden. Wird dem Gassammelraum weniger Gas entnommen als zugeführt, strömt es über die Unterkante des Gassammelraums und gelangt in den darüber angeordneten Trichter, der das Gas durch ein Rohr nach oben weitergibt. Auf diese Weise entstehen seitlich über dem Doppeltrichter gasarme Räume, in denen die Biomasse sedimentieren kann. Dieser bekannte Biogasreaktor hat jedoch grundsätzliche Nachteile, die sich als für die technische Anwendung negativ herausgestellt haben.

Im Gassammelraum wird, da der gesamte Trichter mit Gas gefüllt ist, ein relativ großes Gasvolumen aufgestaut. Da in diesem Volumen keine Reaktion stattfindet, geht dieser Raum für die Umsetzungsvorgänge im Biogasreaktor verloren. Unter der Voraussetzung, daß die durch die Doppeltrichteranordnung gebildeten Trennelemente in bestimmten fest vorgegebenen Abständen übereinander angeordnet werden, wobei geeignet ein Abstand im Bereich von 1-5 m ist, nimmt das Volumen der Gassammelräume mit zunehmendem Reaktordurchmesser einen immer größeren Anteil des Reaktorgesamtvolumens ein. Bei einem Reaktordurchmesser von z.B. 4 m und einem Öffnungswinkel des Auffangtrichters von 90° ergibt sich bereits für jedes Trennelement ein Gassammelraumvolumen von etwa 4-5 m³. Ein Gasvolumen dieser Größe erzeugt einen erheblichen Auftrieb, der nur durch konstruktiv aufwendige Maßnahmen beherrscht werden kann. Die technisch denkbare Aufteilung eines großen Doppeltrichters in mehrere nebeneinander angeordnete kleinere, eventuell in prismatischer Form, verringert zwar den Auftrieb, führt aber auch zu einer Erhöhung des konstruktiven Aufwandes.

Ein weiterer Nachteil des bekannten Biogasreaktors besteht darin, daß zwischen dem aus dem Sedimentationsraum nach unten abfließenden Sediment-Schlamm und der aufwärts gerichteten Strömung keine klare Trennung erfolgt.

Nach der EP-A-0 142 307 ist ferner eine Vorrichtung zur Behandlung von Klärschlämmen bekannt, in deren Behälter ein mit der Spitze nach unten gerichteter Trichter angeordnet ist. In dem Trichter ist eine vertikale Trennwand ausgebildet, die eine Klärschlammvorlaufkammer und eine Rezirkulationskammer bildet. In die Klärschlammvorlaufkammer wird Klärschlamm eingeführt, der durch ein mittels eines Ventils verschließbares Rohr am unteren Bereich des zugehörigen Trichterabschnittes in den unteren Sedimentationsraum des Behälters eintritt. Eingedickter Klärschlamm wird am Boden des Behälters abgeführt. Faulgase steigen in einen Gassammelraum auf, der an der Unterseite des Trichters durch die Behälterwand und einen vertikalen Trennwandabschnitt gebildet ist. Über ein Gasabzugsrohr können die Faulgase abgezogen werden. Ein Teil der Faulgase kann auch über die untere Kante des Trennwandabschnitts in die Ausströmkammer gelangen. In dieser befindliche Klärschlammsuspension wird durch ein an dem Trichter angeordnetes weiteres Rohr wieder in den unteren Sedimentationsraum des Behälters eingebracht. Diese Vorrichtung ermöglicht nur eine Klärschlammbehandlung in einer Stufe, nicht aber eine Ausbildung mehrerer Sedimentationszonen in verschiedenen Reaktorhöhen.

Die Aufgabe der Erfindung besteht darin, den Biogasreaktor der eingangs genannten Art so zu verbessern, daß er eine einfache säulenartige Bauform mit geringer Grundfläche aufweist, wobei bei großer Bauhöhe die Auftriebskräfte vernachlässigbar sein sollen, definierte Sedimentationszonen geschaffen und eine durch eine ausreichende Umlaufströmung bewirkte Durchmischung des Reaktorinhalts erzielt werden und Biogas aus allen - auch tieferen - Reaktorzonen abziehbar sein soll.

Erfindungsgemäß erfolgt die Lösung der Aufgabe durch die kennzeichnenden Merkmale des Anspruchs 1. Vorteilhafte Ausgestaltungen der Erfindung werden in den abhängigen Ansprüchen beschrieben.

Die Erfindung wird im folgenden am Beispiel der in den Zeichnungen dargestellten Ausführungsformen näher erläutert. Es zeigt in schematischen Seitenansichten

Fig. 1
eine Ausbildung eines Trennelements,

Fig. 2 und 3
weitere Ausbildungen von Trennelementen,

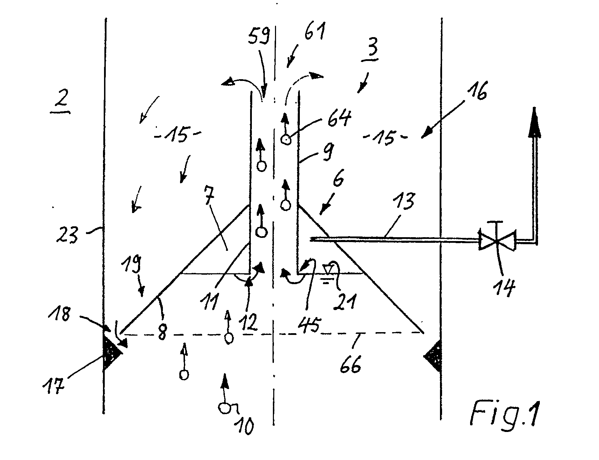

Fig. 4
eine Ausführung eines Biogasreaktors mit kaskadenartig übereinander angeordneten Trennelementen nach Fig. 1,

Fig. 5 und 6
die Ausbildung von Flüssigkeitsfüllstandsregeleinrichtungen an Trennelementen nach Fig. 1,

Fig. 7a und 7b
eine Ausführung eines Biogasreaktors mit kaskadenartig und parallel zueinander angeordneten Trennelementen.

Fig. 1 zeigt ein Trennelement 3, das von der Wand 23 eines Reaktorgehäuses 2 umgeben ist. Das Trennelement 3 besteht aus einem mittigen Rohr 9 an dem ein sich unten öffnender Trichter 8 angeformt ist. Das Rohr 9 ist so mit dem Trichter 8 verbunden, daß ein Rohrabschnitt 11 in den Trichterraum hineinragt. Der freie Endabschnitt 45 des Rohrabschnitts 11 ist bis zum Flüssigkeitsspiegel 21 geführt. Oberhalb des Flüssigkeitsspiegels 21 befindet sich der durch den Rohrabschnitt 21 und einen Abschnitt des Trichters 8 gebildete Gassammelraum 7. Am Endabschnitt 19 des Trichters 8 ist unter Ausbildung eines Spaltes 18 eine Gasabweiseinrichtung 17 angeordnet, die z. B. als Leitring ausgebildet sein kann. Der oberhalb des Trichters 8 ausgebildete Gehäuseabschnitt 15 dient als Sedimentationszone 16. Der Gassammelraum 7 ist mit einer Leitung 13 verbunden, die aus dem Reaktorgehäuse 2 herausgeführt ist. In der Leitung 13 befindet sich ein Ventil 14.

Die Überströmkante 12 des Rohrabschnitts 11 ist in dem Trichter 8 im Abstand von der durch den freien Endabschnitt 19 des Trichters 8 gebildeten horizontalen Ebene 66 angeordnet.

Bei dem Trennelement 3 dient somit nicht das gesamte Volumen des Trichters 8 als Gassammelraum 7 sondern nur ein Teil. Das aus der Flüssigkeit aufsteigende Gas 10 wird im Gassammelraum 7 aufgestaut und kann über die Leitung 13 nach außen abgeführt werden. Überschüssiges Gas strömt über die Überströmkante 12 des Rohrabschnitts 11 in das offene Rohr 9, das einen Gasabzugskanal 59 bildet. Durch die in dem Rohr 9 aufsteigenden Gasblasen 64 wird nach dem Mammutpumpenprinzip eine nach oben gerichtete Strömung erzeugt. Gleichzeitig entsteht im seitlich über dem Trichter 8 befindlichen Gehäuseabschnitt 15 aus Kontinuitätsgründen eine abwärts gerichtete Strömung. Da dieser Gehäuseabschnitt 15 gegenüber aus unteren Regionen aufsteigendem Gas durch die Gasabweiseinrichtung 17 abgeschirmt ist, wird im Bereich des Gehäuseabschnitts 15 eine Zone mit günstiger Sedimentationseigenschaft erzeugt. Es ist auch möglich, den Trichter des Trennelements mit nach unten gerichteter Spitze anzuordnen. In diesem Fall ist der Trichter randseitig von einem Rohr zu umgeben, daß im Abstand zur Wand des Reaktorgehäuses angeordnet wird. Hierdurch wird ein als Gasabzugskanal dienender Ringkanal gebildet, wobei durch den unteren Abschnitt des Rohres ebenfalls der Gassammelraum 7 definiert wird. An der Spitze des Trichters kann eine Durchbrechung mit einem davor befindlichen Führungskörper vorgesehen sein. Durch den so gebildeten Spalt kann aus dem Sedimentationsraum Fluid nach unten abfließen.

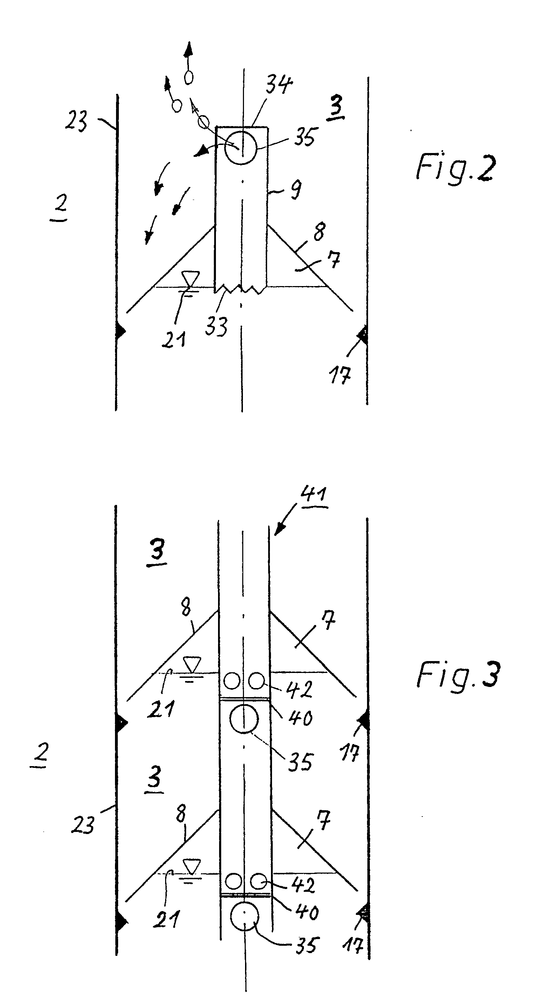

Um eine schwallartige Entladung des jeweiligen Gassammelraums 7 zu vermeiden, können an den Überströmkanten 12 Kerben 33 vorgesehen werden (Fig. 2). Um zu verhindern, daß aus einem Rohr 9 eines Trennelements 3 austretende Gase direkt in ein weiteres Rohr 9 eines darüber befindlichen Trennelements 3 eintreten, ist es möglich, am freien Endabschnitt der Rohre 9 der Trennelemente 3 jeweils eine kreisringförmigen Verschlußplatte 34 vorzusehen. In diesem Fall ist es erforderlich unter der Verschlußplatte in dem Rohr 9 mindestens eine Durchbrechung 35 als Austrittsöffnung vorzusehen (Fig. 2).

Fig. 3 zeigt eine Ausführungsform, bei der die Rohre 9 von Trennelementen 3 eines Biogasreaktors 1 zu einem zentralen Leitrohr 41 verbunden sind. In diesem Fall muß in dem Leitrohr 41 zwischen den Trennelementen 3 jeweils eine Trennwand 40 als Strömungssperre angeordnet werden. Oberhalb jeder Trennwand 40 aber unterhalb des jeweiligen Flüssigkeitsspiegels 21 befinden sich in dem Leitrohr 41 Durchbrechungen 42 als Gaseintrittsöffnungen. Unterhalb jeder Trennwand 40 ist mindestens eine Durchbrechung 35 als Gasaustrittsöffnung ausgebildet.

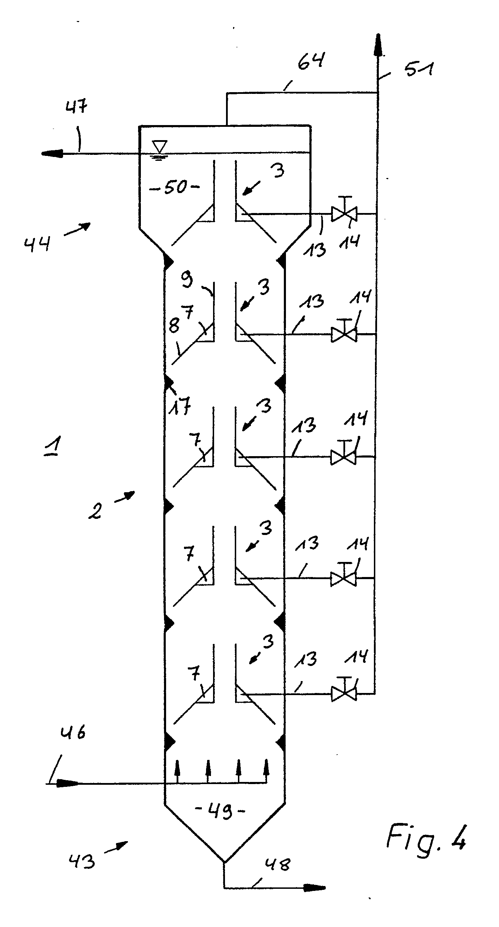

Fig. 4 zeigt einen Biogasreaktor 1 in dem mehrere Trennelemente 3 übereinander angeordnet sind. Am unteren Abschnitt 43 des Reaktorgehäuses 2 ist ein Sedimentationsraum 49 ausgebildet, der an seiner tiefsten Stelle mit einer Abflußleitung 48 verbunden ist. Ferner ist im Bereich des unteren Abschnitts 43 ein Abwasserzuführstutzen 46 vorgesehen. Der obere Abschnitt 44 des Reaktorgehäuses 2 ist ebenfalls als Sedimentationsraum 50 ausgebildet. Dieser Abschnitt ist im Durchmesser größer gestaltet als der darunter befindliche Gehäuseabschnitt des Reaktorgehäuses 2. Der obere Abschnitt 44 des Reaktorgehäuses 2 weist ferner einen Abflußstutzen 47 sowie eine Gasleitung 64 auf. Jeder Gassammelraum 7 ist mittels einer Leitung 13 mit Ventil 14 mit einer zentralen Gasleitung 51 verbunden, an die auch die bereits erwähnte Gasleitung 64 angeschlossen ist. Es ist aber auch möglich, nur einzelne Gassammelräume 7 mittels der Leitung 13 mit der zentralen Gasleitung 51 zu verbinden, sofern dies bei dem vorgesehenen Prozeß im Biogasreaktor 1 erforderlich oder ausreichend ist.

Der in Fig. 4 dargestellte Biogasreaktor 1 kann Reaktorhöhen zwischen 5 und ca. 100 Metern aufweisen. Das zu vergärende Abwasser wird dem Biogasreaktor 1 durch den Abwasserzuführstutzen 46 zugeführt. Der Sedimentationsraum 49 im unteren Abschnitt 43 des Reaktorgehäuses 2 dient der Anreicherung von Überschußschlamm sowie von inerten, biologisch nicht aktiven Feststoffen, die dann über die Abflußleitung 48 abgezogen werden können. Im oberen Abschnitt 44 des Biogasreaktors 1 wird die Sedimentation und mechanische Klärung des abzuführenden gereinigten Abwassers durch die Vergrößerung des Reaktordurchmessers gefördert. Das hier anfallende Biogas wird über die Gasleitung 64 der zentralen Gasleitung 51 zugeführt.

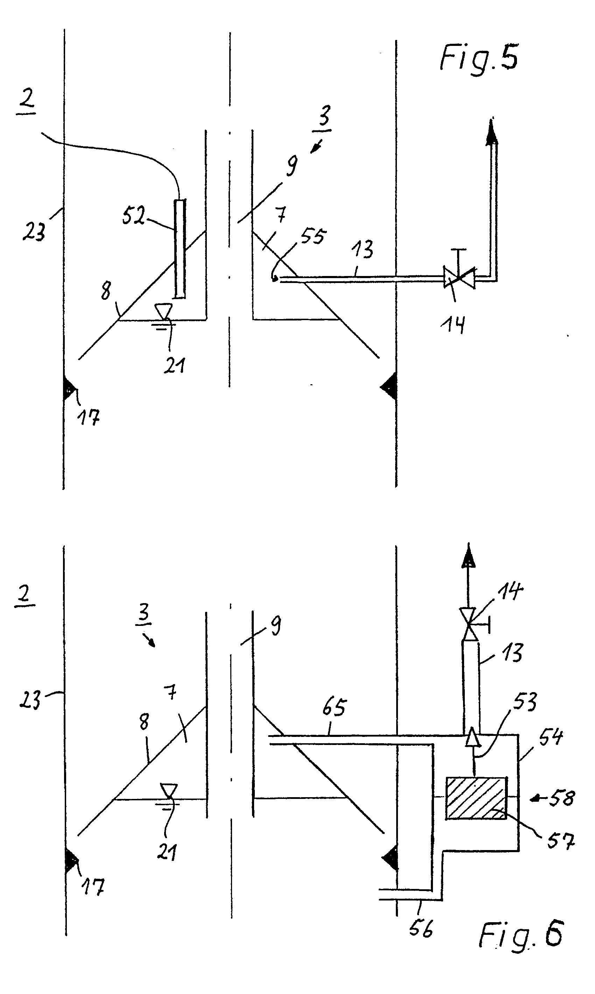

Es ist möglich, einem oder mehreren Gassammelräumen 7 eine Flüssigkeitsfüllstandsregeleinrichtung zuzuordnen um den Betrieb des Biogasreaktors 1 regeln zu können. Eine Flüssigkeitsfüllstandsregeleinrichtung kann z. B. eine Standsonde 52 aufweisen, die in den Gassammelraum 7 geführt ist und über ein Steuergerät mit einem als Regelventil ausgebildeten Ventil 14 in Verbindung steht. Das Ventil 14 ist wie bei dem Biogasreaktor 1 nach Fig. 4 in der Leitung 13 angeordnet, deren Eintrittsöffnung 55 dem betreffenden zu regelnden Gassammelraum 7 zugeordnet ist (Fig. 5). Wenn mittels der Standsonde 52 eine Anhebung des Flüssigkeitsspiegels 21 festgestellt wird, wird das Ventil 14 so eingestellt, daß das in dem Gassammelraum 7 befindliche Gas den Flüssigkeitsspiegel 21 wieder nach unten drückt (Fig. 5).

Eine weitere Flüssigkeitsfüllstandsregeleinrichtung ist in Fig. 6 dargestellt. Diese ist als Schwimmerventil 58 ausgebildet. Es besteht aus einem Schwimmergehäuse 54, das mittels eines Anschlußrohrs 56 mit dem Flüssigkeitsraum des jeweiligen Trennelements und mittels einer Leitung 65 mit dem Gassammelraum 7 verbunden ist. An das Schwimmergehäuse 54 ist eine Leitung 13 mit einem Ventil 14 angeschlossen. In dem Schwimmergehäuse 54 befindet sich ein Schwimmer 57. Der Flüssigkeitsstand im Schwimmergehäuse 54 entspricht dem Stand des Flüssigkeitsspiegels 21. An dem Schwimmer 57 ist ein Ventilkörper 53 ausgebildet, dem die Einlaßöffnung des Rohrs 13 als Ventilsitz zugeordnet ist. Der Ventilkörper 53 kann einstellbar ausgebildet sein, z.B. durch Verstellung des Schwimmers 57.

Für den Betrieb des Biogasreaktors 1 sind zwei Extremzustände denkbar, die durch die Menge des abzuleitenden Biogases festgelegt werden. Soll das gesamte aus den unteren Reaktorzonen aufsteigende Gas nach außen abgeleitet werden, so kann das dadurch geschehen, daß die Ventile 14 vollkommen geöffnet werden. In diesem Fall wird bei ausreichendem Gasabfluß der Flüssigkeitsspiegel 21 bis zur Eintrittsöffnung 55 der jeweiligen Leitung 13 ansteigen. Hierdurch wird neben Gas auch Flüssigkeit in die Leitungen 13 gelangen und über die zentrale Gasleitung 51 abgeführt werden. An anderer Stelle muß dann die Flüssigkeit wieder vom Gas getrennt werden. Das Eindringen von Flüssigkeit in die Leitungen 13 kann durch Verwendung von Flüssigkeitsfüllstandsregeleinrichtungen vermieden werden wie sie beispielsweise oben beschrieben und in den Fig. 5 und 6 schematisch dargestellt sind. Ein Reaktorbetrieb mit einer maximalen Gasabfuhr, bei dem die Sedimentation bevorzugt wird, wird regelmäßig vorzugsweise in den oberen Reaktorzonen angebracht sein. In den unteren Reaktorzonen kann zur Erzielung einer guten Substrateinmischung ein weiterer extremer Betriebszustand erforderlich sein, bei dem das gesamte aus den unteren Reaktorzonen aufsteigende Gas durch die Rohre 9 der jeweiligen Trennelemente 3 strömt. Dieser Zustand wird durch Schließen der Ventile 14 erzielt.

Während bei der erstgenannten Betriebsweise durch Unterdrückung der Mammutpumpenwirkung eine weitgehende Beruhigung der Strömung erzielt wird und für die aktive Biomasse günstige Sedimentationsbedingungen entstehen, begünstigt die zweitgenannte Betriebsweise eher die Durchmischung. Aufgrund der starken Rückvermischung zwischen aufwärts und abwärts gerichteter Strömung wird die Wirkung der Sedimentation weitgehend unterdrückt.

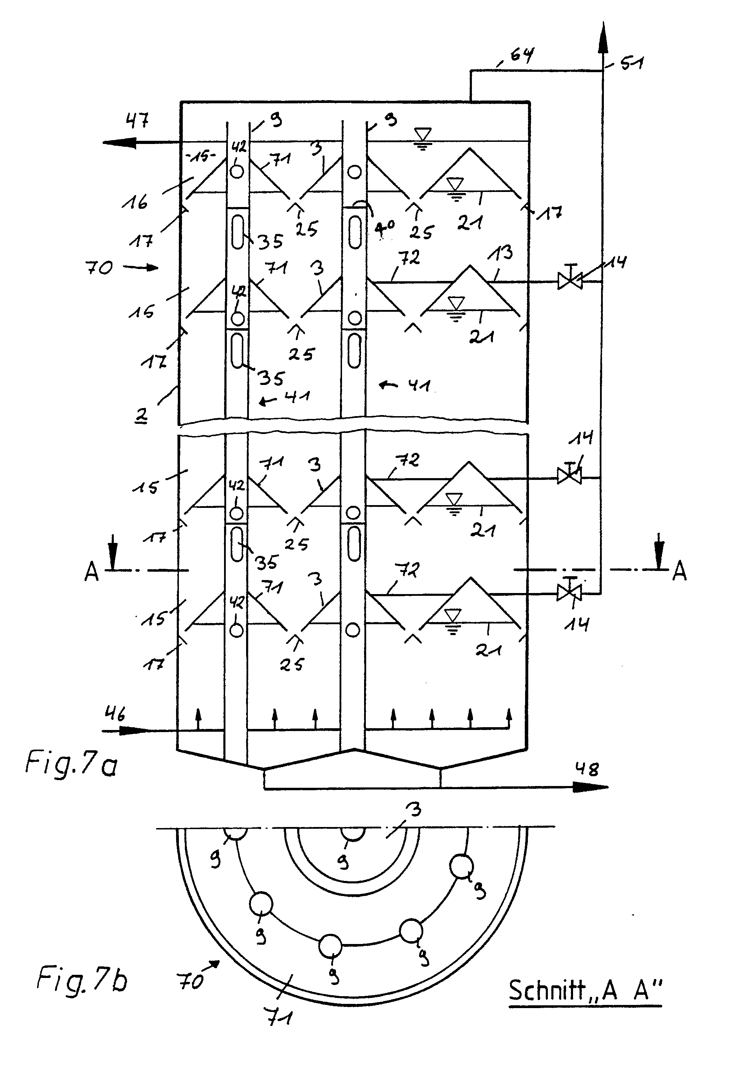

In den Figuren 7a und 7b ist eine weitere mögliche Großausführung eines Biogasreaktors 70 dargestellt. Geeignete Durchmesser dieses Biogasreaktors 70 liegen im Bereich von 5 bis 15 m und betragen vorzugsweise ca. 9 m. Die Reaktorhöhe liegt zwischen 15 und 50 m und beträgt vorzugsweise 30 m. In dem Reaktorgehäuse 2 des Biogasreaktors 70 sind in vertikalem Abstand voneinander Trennelemente 3, 71 angeordnet. Das zentrale Trennelement 3 hat die Form eines Kreiskegels wie in Fig. 1 dargestellt. Das Trennelement 71 ist kreisförmig ausgebildet und weist einen dreieckigen Querschnitt auf, stellt also ein gebogenes Dreieckprisma dar. Die Trennelemente 3, 71 werden von senkrecht ausgerichteten Rohren 9 gehalten, die zu zentralen Leitrohren 41 verbunden sind. Die Rohre 9 bzw. Leitrohre 41 der Trennelemente 71 sind kreisringförmig angeordnet (Fig. 7b). Die Rohre 9 weisen Durchbrechungen 42 zur Aufnahme des produzierten Biogases bzw. von Gärsuspension auf, die innerhalb der Rohre 9 durch das aufsteigende Biogas nach oben gepumpt wird. Durch die Durchbrechungen 35 in den Rohren 9 wird das Gas-Flüssigkeitsgemisch wieder in den außerhalb der Rohre 9 liegenden Reaktorraum abgeführt. Zwischen den Durchbrechungen 35 und 42 sind die Rohre 9 jeweils durch eine Trennwand 40 verschlossen. Den unteren Trennelementen 3, 71 sind nur Durchbrechungen 42 zugeordnet. Trennwände 40 und Durchbrechungen 35 entfallen hier.

Die den obersten Trennelementen 3, 71 zugeordneten Durchbrechungen 42 sind im Vergleich zu den Durchbrechungen 42 der übrigen Trennelemente 3, 71 höher angeordnet und befinden sich im Bereich der Gassammelräume 7 der obersten Trennelemente 3, 71. Durch diese Durchbrechungen 42 wird ausschließlich Gas, jedoch keine Gärsuspension geleitet. Der Flüssigkeitsspiegel 21 wird durch den Entnahmedruck für das Biogas vorgegeben, das durch die zentrale Gasleitung 51 als Entnahmeleitung strömt. Die Flüssigkeitsspiegel 21 der darunter liegenden Trennelemente werden entweder durch den Öffnungszustand der Ventile 14 bzw. als untere Niveaubegrenzung durch die den Durchbrechungen 42 entsprechenden horizontalen Ebenen festgelegt. Die Trennelemente 3, 71 einer Kaskade sind durch jeweils mindestens eine Gasleitung 72 miteinander verbunden. Von den Trennelementen 71 wird das entstandene Biogas durch Leitungen 13 mit Ventilen 14 der zentralen Gasleitung 51 zugeführt. Um aufsteigende Gasblasen von den Sedimentationszonen 16 in den Gehäuseabschnitten 15 fernzuhalten, sind Gasabweiseinrichtungen 17 bzw. Führungskörper 25 vorgesehen. Dem Biogasreaktor 70 wird frisches Substrat über den Abwasserzuführstutzen 46 zugeführt. Die gereinigte Flüssigkeit wird über den Abflußstutzen 47 dem Biogasreaktor 70 entnommen. Für die Entnahme von Schlamm bzw. inertem sedimentierten Material dient die Abflußleitung 48. Es ist auch möglich, statt eines Trennelements 71 mehrere Trennelemente 71 in jeder Kaskade vorzusehen, wobei die einzelnen Trennelemente dann unterschiedliche Durchmesser aufweisen. Auch in diesem Fall werden Gasleitungen 72 vorgesehen, um das entstehende Biogas von dem Trennelement 3 und den inneren Trennelementen 71 über das äußere Trennelement 71 abführen zu können. Die Rohre 9 bzw. die zentralen Leitrohre 41 werden jeweils so angeordnet, daß sie einen etwa gleichen Einzugsbereich haben, damit der Biogasreaktor 70 gleichmäßig betrieben werden kann.

Es sind verschiedene Ausführungen von Biogasreaktoren 1, 70 möglich. So können die Trichter 8 der Trennelemente 3 eine prismatische, pyramiden- oder kegelartige Form haben. Es ist auch möglich, unterschiedliche dieser Trennelemente 3 in einem Biogasreaktor 1, 70 miteinander zu kombinieren. Durch entsprechende Einstellung der Ventile 14 können die Trennelemente 3, 71 in den einzelnen Reaktorzonen des Biogasreaktors 1, 70 so eingestellt werden, daß eine möglichst gleichmäßige Verteilung der aktiven Biomasse innerhalb des Reaktors erzielt wird, wobei der Bereich des Abflußstutzens 47 frei von Biomasse zu halten ist. Sofern eine verstärkte Durchmischung des Biogasreaktors 1, 70 gewünscht wird können die Ventile 14 in den unteren Reaktorzonen gedrosselt werden oder aber es wird in diesem Bereich durch Fortfall der Gasentnahmeleitungen 13 und Ventile 14 auf eine Gasentnahme verzichtet.

## Patentansprüche

1. Biogasreaktor, dessen Gehäuse (2) ganz - oder teilweise mit aktiver Biomasse gefüllt ist, in dem mittels eines oder mehrerer säulenartig übereinander angeordneter sich jeweils nach unten erweiternder trichterförmiger Trennelemente in deren Reaktorabschnitten jeweils ein Gassammelraum (7) zum Sammeln der aus der Gärsuspension aufsteigenden Gase und ein gasblasenarmer Sedimentationsraum zur Sedimentation aktiver Biomasse - Schlammteilchen ausgebildet ist, in den eine Leitung (13) mit einem Drosselorgan (14) zur Gasabführung geführt ist, mit einer am trichterförmigen Trennelement ausgebildeten Überströmkante (12) und einem an der Trennelementspitze angeordneten rohrartigen Kanalstück, das sich bis in den oberhalb des trichterförmigen Trennelements befindlichen Reaktorraum erstreckt, dadurch gekennzeichnet, daß die Trichter (8) der Trennelemente (3) derart mit einem als Rohr (9) ausgebildeten Gasabzugskanal (59) verbunden sind, daß der dem Flüssigkeitsspiegel (21) zugewandte Rohrabschnitt (11) in den Trichter (8) hineinragt, mit einem Abschnitt des Trichters (8) den Gassammelraum (7) und am freien Endabschnitt (45) des Rohrabschnitts (11) die Überströmkante (12), für den Gasabzugskanal (59) bildet, wobei die Überströmkante (12) des Rohrabschnitts (11) in dem Trichterinnenraum im vertikalen Abstand von der durch den unteren freien Endabschnitt (19) des Trichters (8) gebildeten horizontalen Ebene angeordnet ist.

2. Biogasreaktor nach Anspruch 1, dadurch gekennzeichnet, daß in den Überströmkanten (12) der Rohrabschnitte (11) Kerben (33) ausgebildet sind.

3. Biogasreaktor nach Anspruch 1 und 2, dadurch gekennzeichnet, daß der obere Endabschnitt (61) des Rohrs (9) mit einer Verschlußplatte (34) verschlossen ist, unter der in dem Rohr (9) mindestens eine Durchbrechung (35) als Gasaustrittsöffnung ausgebildet ist.

4. Biogasreaktor nach Anspruch 1, dadurch gekennzeichnet, daß die Rohre (9) der Trichter (8) der Trennelemente (3) zu einem zentralen Leitrohr (41) verbunden sind, wobei in dem Leitrohr (41) zwischen den Trennelementen (3) als Strömungssperre jeweils eine Trennwand (40) angeordnet ist und oberhalb jeder Trennwand (40) im Bereich des Trichters (8) des betreffenden Trennelements (3) mindestens eine den Gassammelraum (7) definierende Durchbrechung (42) als Überströmkante für den Gaseintritt und unterhalb jeder Trennwand (40) mindestens eine Durchbrechung (35) als Öffnung für den Gasaustritt ausgebildet ist.

5. Biogasreaktor nach einem der Ansprüche 1 bis 4, bei dem jeder Gassammelraum (7) mit einer Flüssigkeitsfüllstandsregeleinrichtung verbunden ist, dadurch gekennzeichnet, daß die Flüssigkeitsfüllstandsregeleinrichtung eine in den Gassammelraum (7) geführte Standsonde (52) aufweist, die über ein Steuergerät mit einem als Regelventil ausgebildeten Ventil (14) in Verbindung steht, das in der Leitung (13) angeordnet ist, deren Eintrittsöffnung (55) dem betreffenden Gassammelraum (7) zugeordnet ist.

6. Biogasreaktor nach einem der Ansprüche 1 bis 4, bei dem jeder Gassammelraum (7) mit einer Flüssigkeitsfüllstandsregeleinrichtung verbunden ist, dadurch gekennzeichnet, daß die Flüssigkeitsfüllstandsregeleinrichtung als Schwimmerventil (58) mit ggf. einstellbarem Ventilkörper (53) ausgebildet ist, das mit dem Gassammelraum (7) und der Flüssigkeit unter dem Gassammelraum (7) verbunden ist.

7. Biogasreaktor nach Anspruch 1 bis 4, dadurch gekennzeichnet, daß mittig in dem Reaktorgehäuse (2) übereinander Trennelemente (3) mit einem zentralen Leitrohr (41) angeordnet sind, die kreisförmig von mindestens jeweils einem weiteren Trennelement (71) umgeben sind, dessen Querschnitt dem Querschnitt der Trennelemente (3) entspricht und durch das kreisförmig angeordnete Rohre (9) geführt sind, die jeweils zu zentralen Leitrohren (41) verbunden sind.

**Claims**

1. Biogasreactor, the housing (2) of which is total or partly filled with active biomass having one or more partition elements which are arranged columnlike one upon another, each partition element is funnel-shaped and towards below widened in the reactor segments of which in each case is a gas collecting room (7) for collecting gases which rises from the ferment suspension and a sedimentation room being poor of gas bubbles for sedimentation of active biomass mud particles into which a conduit-pipe (13) with a throttle device (14) for diversion of gas is led, whereby the funnel-shaped partition element has an overflow edge (12) and a pipelike channel piece at the peak of the partition element, which extends into the reactor room being above of the funnel-shaped partition element, characterized in that the funnels (8) of partition elements (3) are connected with a pipe (9) being a gasoutlet channel (59) in such a way that the pipe segment (11) turned to the liquid level (21) looms into the funnel (8) forming the gas collecting room (7) and at the free end piece (45) of pipe segment (11) the overflow edge (12) for the gas outlet channel (59), whereby the overflow edge (12) of pipe segment (11) inside of the funnel (8) is arranged in a vertically distance to the horizontal level being formed by the downside free endpiece (19) of funnel (8).

2. Biogasreactor according to claim 1, characterized in that at the overflow edges (12) of pipe segments (11) notches (33) are developed.

3. Biogasreactor according to claim 1 and 2, characterized in that, the superior end piece (61) of pipe (9) is closed by a fastening plate (34) under which at least one penetration hole (35) is formed being a gasoutlet opening.

4. Biogasreactor according to claim 1, characterized in that, pipes (9) of funnels (8) of partition elementes (3) are combined to a central guard pipe (41), whereby within the guard pipe (41) and between the partition elements (3) a partition wall (40) is arranged as current shutting and above each partition wall (40) in the area of the funnel (8) concerning to the partition element (3) at least one throughhole (42) is formed as overflow edge for gas entrance defining the gas collecting room (7) and below each partition wall (40) at least one throughole (35) is formed being an opening for the gas outlet.

5. Biogasreactor according to one of claims 1 to 4, at which each gas collecting room (7) is connected with a device for controlling the level of liquid, characterized in that, the device for controlling the level of liquid has a stand sonde (52) which is led into the gas collecting room (7) which is in connection with a valve (14) being a control valve which is connected to a control device , whereby the valve (14) is arranged within a pipe (13) the inlet opening (55) of which is allocated to the concerning gas collecting room (7).

6. Biogasreactor according to one of the claims 1 to 4, at which each of the gas collecting rooms (7) in combined with a device for controlling the level of liquid, characterized in that, the device for controlling the level of liquid is constructed as float valve (58) with an adjustable valve body (53) if need be, which is combined with the gas collecting room (7) and the liquid below the gas collecting room (7).

7. Biogasreactor according to claims 1 to 4, characterized in that in the middle of the housing (2) of the reactor are partition elements (3) arranged one upon another with a central guiding pipe (41), which are surrounded by at least one another partition element (3) the cross-section of which is according to the cross-sections of the partition elements (3) through which circular arranged pipes (9) are led, which are combined to central guiding pipes (41).

**Revendications**

1. Réacteur à biogaz dont l'enveloppe (2) est remplie entièrement ou partiellement de biomasse active, dans lequel, au moyen d'un ou de plusieurs éléments de séparation en forme de cônes s'évasant chacun vers le bas et disposés l'un au-dessus de l'autre en colonnes ainsi que dans leurs segments de réacteur, est formé chaque fois un espace de collecte de gaz (7) destiné à recueillir les gaz se dégageant de la suspension fermentante, avec un espace de sédimentation sans bulles de gaz pour la sédimentation de particules de biomasse active-boues, dans lequel s'ouvre une conduite (13) pourvue d'une valve d'étranglement (14) pour l'évacuation du gaz, comportant une arête d'écoulement (12) formée sur l'élément de séparation en forme de cône et un bout de conduit tubulaire disposé dans la pointe de l'élément de séparation, qui s'étend jusque dans l'espace du réacteur se trouvant au-dessus de l'élément de séparation en forme de cône, caractérisé en ce que les cônes (8) des éléments de séparation (3) sont reliés à un conduit d'évacuation de gaz (59) ayant la forme d'un tube (9) d'une manière telle que le segment de tube (11) tourné vers le niveau de liquide (21) s'étende dans le cône (8), forme, avec un segment du cône (8), l'espace de collecte de gaz (7) et, à l'extrémité libre (45) du segment de tube (11), l'arête d'écoulement (12) pour le conduit d'évacuation de gaz (59), l'arête d'écoulement (12) du segment de tube (11) étant disposée dans l'espace intérieur du cône, à une distance verticale du plan horizontal formé par la section d'extrémité inférieure libre (19) du cône (8).

2. Réacteur à biogaz suivant la revendication 1, caractérisé en ce que des entailles (33) sont ménagées dans les arêtes d'écoulement (12) des segments de tube (11).

3. Réacteur à biogaz suivant les revendications 1 et 2, caractérisé en ce que le segment d'extrémité supérieur (61) du tube (9) est fermé par une plaque d'obturation (34) en dessous de laquelle, dans le tube (9), au moins une ouverture (35) est prévue à titre d'ouverture de sortie de gaz.

4. Réacteur à biogaz suivant la revendication 1, caractérisé en ce que les tubes (9) des cônes (8) des éléments de séparation (3) sont raccordés en un conduit central (41), une cloison de séparation (40) étant disposée dans le conduit (41) à titre d'arrêt d'écoulement, chaque fois entre les éléments de séparation (3) et, au-dessus de chaque cloison de séparation (40), dans la zone du cône (8) de l'élément de séparation (3) en question, au moins une ouverture (42) définissant l'espace de collecte de gaz (7) est ménagée à titre d'arête d'écoulement pour l'entrée du gaz et, en dessous de chaque cloison de séparation (40), au moins une ouverture (35) est ménagée à titre d'ouverture pour la sortie du gaz.

5. Réacteur à biogaz suivant l'une quelconque des revendications 1 à 4, dans lequel chaque espace de collecte de gaz (7) est raccordé à un dispositif de réglage de l'état de remplissage de liquide, caractérisé en ce que le dispositif de réglage de l'état de remplissage de liquide comporte une sonde fixe (52) qui est en communication, par l'intermédiaire d'un appareil de commande, avec une valve (14) ayant la forme d'une valve de réglage branchée dans la conduite (13) et dont l'orifice d'entrée (55) se trouve dans l'espace de collecte de gaz (7) associé.

6. Réacteur à biogaz suivant l'une quelconque des revendications 1 à 4, dans lequel chaque espace de collecte de gaz (7) est raccordé à un dispositif de réglage de l'état de remplissage de liquide, caractérisé en ce que le dispositif de réglage de l'état de remplissage de liquide a la forme d'une valve à flotteur (58) comportant un obturateur (53), le cas échéant réglable, qui est raccordé à l'espace de collecte de gaz (1) et au liquide présent en dessous de l'espace de collecte de gaz (7).

7. Réacteur à biogaz suivant l'une quelconque des revendications 1 à 4, caractérisé en ce qu'au centre de l'enveloppe (2) du réacteur sont disposés des éléments de séparation superposés (3) avec un conduit central (41), qui sont entourés en cercle chaque fois par au moins un autre élément de séparation (71) dont la section transversale correspond à celle des éléments de séparation (3) et qui est traversé par des tubes (9) disposés en cercle, qui sont chaque fois raccordés en un conduit central (41).

Fig.1

Fig.2

Fig.3

Fig. 4

Fig.5

Fig.6

EP 0 300 348 B1

Fig.7a

Fig.7b

Schnitt "A A"

13